# EUROPÄISCHE PATENTSCHRIFT

(11) **EP 0 648 767 B1**
(45) Veröffentlichungstag und Bekanntmachung des Hinweises auf die Patenterteilung: **28.05.1997**
(21) Anmeldenummer: 94114798.5
(22) Anmeldetag: 20.09.1994
(51) Int. Cl.: C07D 405/14, A61K 31/495

(54) **Piperidine und Piperazine, die Wirkungen auf das z.n.s. Zeigen**
Piperdine and piperazine derivatives which affect the C.N.S.
Dérivés de piperidine et piperazine qui puissent agir sur le "CNS"

(30) Priorität: 30.09.1993 DE 4333254
(43) Veröffentlichungstag der Anmeldung: 19.04.1995
(73) Patentinhaber: MERCK PATENT GmbH, 64293 Darmstadt (DE)
(72) Erfinder: Böttcher, Henning, Dr., D-64287 Darmstadt (DE); Seyfried, Christoph, Dr,, D-64342 Seeheim-Jugenheim (DE); Bartoszyk, Gerd, D-64289 Darmstadt (DE); Greiner, Hartmut, Dr., D-64287 Darmstadt (DE)

(56) Entgegenhaltungen:
- EP-A- 0 490 772
- WO-A-94/13659
- DE-A- 4 101 686
- DE-A- 4 127 849
- FR-A- 1 551 082
- GB-A- 1 075 156

## Beschreibung

Die Erfindung betrifft neue Piperidin- und Piperazinderivate der Formel I 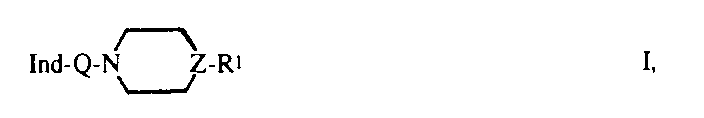 worin
- Ind: einen unsubstituierten oder einen ein- oder zweifach durch OH, OA, CN, Hal, COR² oder CH₂R² substituierten Indol-3-yl-rest,
- R¹: unsubstituiertes oder einfach durch CN, CH₂OH, CH₂OA oder COR² substituiertes Benzofuran-5-yl bzw. 2,3-Dihydrobenzofuran-5-yl, Chroman-6-yl, Chroman-4-on-6-yl, 3-Chromen-6-yl oder Chromen-4-on-6-yl,
- Q: CₘH₂ₘ,
- Z: N oder CR³,
- A: Alkyl mit 1-6 C-Atomen,
- Hal: F, Cl, Br oder I,
- R²: OH, OA, NH₂, NHA oder NA₂,
- R³: H, OH oder OA und
- m: 2, 3 oder 4
bedeuten,
sowie deren physiologisch unbedenkliche Salze.

Der Erfindung lag die Aufgabe zugrunde, neue Verbindungen aufzufinden, die zur Herstellung von Arzneimitteln verwendet werden können.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem 5-HT_{1A}-agonistische und 5-HT-Reuptake hemmende Wirkungen. Die Verbindungen zeigen ferner serotonin-agonistische und -antagonistische Eigenschaften. Sie hemmen die Bindung von tritiierten Serotoninliganden an hippocampale Rezeptoren (Cossery et al., European J. Pharmacol. 140 (1987), 143-155). Außerdem treten Veränderungen der DOPA-Akkumulation im Striatum und der 5-HTP-Akkumulation in N. raphe auf (Seyfried et al., European J. Pharmacol. 160 (1989), 31-41). Weiterhin treten analgetische und blutdrucksenkende Wirkungen auf; so wird bei kathetertragenden wachen, spontan hypertonen Ratten (Stamm SHR/Okamoto/NIH-MO-CHB-Kisslegg; Methode vgl. Weeks und Jones, Proc. Soc. Exptl. Biol. Med. 104 (1960), 646-648) der direkt gemessene Blutdruck nach peroraler Gabe der Verbindungen gesenkt. Ebenso eignen sie sich zur Prophylaxe und zur Bekämpfung der Folgen cerebraler Infarktgeschehen (apoplexia cerebri) wie Schlaganfall und cerebraler Ischämien.

Verbindungen der Formel I und ihre physiologisch unbenklichen Säureadditionssalze können daher als Arzneimittelwirkstoffe für Anxiolytika, Antidepressiva, Antipsychotika, Neuroleptika und/oder Antihypertonika und auch als Zwischenprodukte zur Herstellung anderer Arzneimittelwirkstoffe verwendet werden.

Gegenstand der Erfindung sind die Piperidin- und Piperazinderivate der Formel I sowie ihre physiologisch unbedenklichen Säureadditionssalze.

Der Rest A bedeutet Alkyl mit 1, 2, 3, 4, 5 oder 6, insbesondere 1 oder 2 C-Atomen, vorzugsweise Methyl, ferner auch Ethyl, n-Propyl, Isopropyl, n-Butyl, sek.-Butyl oder tert.-Butyl. OA ist vorzugsweise Methoxy, ferner auch Ethoxy, n-Propoxy, Isopropoxy, n-Butoxy, Isobutoxy, sek.-Butoxy oder tert.-Butoxy. NHA ist vorzugsweise Methylamino, ferner Ethylamino, Isopropylamino, n-Butylamino, Isobutylamino, sek.-Butylamino oder tert.-Butylamino. NA₂ bedeutet vorzugsweise Dimethylamino, ferner N-Ethyl-N-methylamino, Diethylamino, Di-n-propylamino, Diisopropylamino oder Di-n-butylamino.

Analog bedeutet CO-NHA vorzugsweise N-Methylcarbamoyl oder N-Ethylcarbamoyl; CO-NA₂ vorzugsweise N,N-Dimethylcarbamoyl oder N,N-Diethylcarbamoyl.

Der Rest Ind bedeutet einen unsubstituierten oder ein- oder zweifach durch einen der angegebenen Reste substituierten Indol-3-ylrest. Vorzugsweise ist er in 5-Stellung, ferner auch in der 4-, 6- oder 7-Stellung substituiert Weiterhin ist eine Substitution in 1- oder 2-Stellung möglich. Bevorzugte Substituenten am Indol-3-ylrest sind OH, OA, CN, CONH₂, CH₂OH, aber auch CO₂H, F, Cl, Br, I, CH₂NH₂, CONHA oder CONA₂, wobei A bevorzugt Methyl oder Ethyl entspricht.

Der Rest R¹ bedeutet vorzugsweise unsubstituiertes oder einfach durch -CH₂OH, -CONH₂, -CO₂A oder -CO₂NHA substituiertes Benzofuran-5-yl, 2,3-Dihydrobenzofuran-5-yl, Chroman-6-yl oder Chromen-4-on-6-yl.

Q ist vorzugsweise -(CH₂)₄-, aber auch -(CH₂)₂- oder -(CH₂)₃-, während Z bevorzugt -N-, -C(OH)- oder -CH- bedeutet.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ig ausgedrückt werden, die der Formel I entsprechen und worin die nicht näher bezeichneten Reste und Parameter die bei der Formel I angegebene Bedeutung haben, worin jedoch
- in Ia Ind: einen in 5-Stellung durch OH oder OA substituierten Indol-3-yl-rest bedeutet;
- in Ib Ind: einen in 5-Stellung durch CONH₂ oder durch CN substituierten Indol-3-yl-rest bedeutet;
- in Ic: Z gleich N ist und R¹ substituiertes oder unsubstituiertes Benzofuran-5-yl bedeutet;
- in Id: Z gleich -C(OH)- ist und R¹ substituiertes oder unsubstituiertes Benzofuran-5-yl bedeutet;
- in Ie: Z gleich N ist und R¹ 2,3-Dihydrobenzofuran-5-yl bedeutet;
- in If: Z gleich N ist und R¹ Chroman-6-yl bedeutet;
- in I g: Z gleich N ist und R¹ Chromen-4-on-6-yl bedeutet.

Insbesondere sind bevorzugt Verbindungen der Teilformeln Ih sowie Iah bis Igh, die den Teilformeln I sowie Ia bis Ig entsprechen, worin jedoch zusätzlich
- Q: -(CH₂)₄- bedeutet.

Benzoderivate sind z.B. bekannt aus der WO 94/13659, 1,4-disubstituierte Piperazinderivate mit 5-HT_{1A} Rezeptoraktivität werden z.B. in der EP 0 490 772 beschrieben. 1,4-Benzodioxanderivate mit Wirkungen auf das Zentralnervensystem sind in der DE 41 27 849 offenbart.

Gegenstand der Erfindung ist ferner ein Verfahren zur Herstellung von Indolderivaten der Formel I sowie von deren Salzen, dadurch gekennzeichnet, daß man eine Verbindung der Formel II

Ind-Q-X¹ II

worin
- X¹: X oder NH₂ und
- X: Cl, Br, I, OH oder eine reaktionsfähig funktionell abgewandelte OH-Gruppe bedeuten und
Ind und Q die angegebenen Bedeutungen haben, mit einer Verbindung der Formel III

X²-(CH₂)₂-ZR¹-(CH₂)₂-X³ III

worin
X² und X³ gleich oder verschieden sein können und, falls X¹ = NH₂ ist, jeweils X, andernfalls zusammen NH bedeuten und
Z und R¹ die angegebenen Bedeutungen haben,
umsetzt
oder daß man zur Herstellung einer Verbindung der Formel I, worin Z gleich N ist, eine Verbindung der Formel IV

Ind-Q-N(CH₂-CH₂-X)₂ IV

worin
X, Q und Ind die angegebenen Bedeutungen haben, mit einer Verbindung der Formel V

R¹-NH₂ V

worin
R¹ die angegebene Bedeutung hat,
umsetzt
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindungen(en) enthält, mit einem reduzierenden Mittel behandelt
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt.
und/oder daß man gegebenenfalls eine OA-Gruppe unter Bildung einer OH-Gruppe spaltet und/oder eine Gruppe Ind und/oder eine Gruppe Ar in eine andere Gruppe Ind und/oder Ar umwandelt und/oder daß man eine erhaltene Base oder Säure der Formel I durch Behandeln mit einer Säure oder Base in eines ihrer Salze umwandelt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York; DE-OS 41 01 686) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Verbindungen der Formel II ist X¹ vorzugsweise X; dementsprechend sind in der Verbindungen der Formel III X² und X³ vorzugsweise zusammen NH. Der Rest X ist vorzugsweise Cl oder Br; er kann jedoch auch I, OH oder eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalinsulfonyloxy).

Dementsprechend sind die Indolderivate der Formel I insbesondere durch Umsetzung von Verbindungen der Formel Ind-Q-Cl oder Ind-Q-Br mit Piperidin/Piperazinderivaten der Formel III, worin X² und X³ zusammen eine NH-Gruppe bedeuten (nachstehend als IIIa bezeichnet) erhältlich.

Die Verbindungen der Formel II und insbesondere III sind zum Teil bekannt; die nicht bekannten Verbindungen der Formeln II und III können leicht analog zu den bekannten Verbindungen hergestellt werden.

Primäre Alkohole der Formel Ind-Q-OH sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Ind-Q-Hal. Die entsprechenden Sulfonyloxyverbindungen sind erhältlich aus den Alkoholen Ind-Q-OH durch Umsetzung mit den entsprechenden Sulfonsäurechloriden.

Die Iodverbindungen der Formel Ind-Q-I sind z.B. durch Einwirkung von Kaliumiodid auf die zugehörigen p-Toluolsulfonsäureester erhältlich. Die Amine der Formel Ind-Q-NH₂ sind z.B. aus den Halogeniden mit Phthalimidkalium oder durch Reduktion der entsprechenden Nitrile herstellbar.

Die Piperazinderivate IIIa sind größtenteils bekannt und z.B. erhältlich durch Umsetzung von Bis-(2-chlorethyl)-amin oder Bis-(2-chlorethyl)-ammonium-chlorid mit 5-Amino-benzofuran, 2,3-Dihydro-5-aminobenzofuran, 6-Aminochroman oder 6-Amino-chromen-4-on oder einem entsprechend substituierten Derivat der genannten Verbindungen. Verbindungen der Formel III (X² und X³ = jeweils X) sind z.B. herstellbar durch Reduktion von Diestern der Formel AlkylOOC-CH₂-ZR¹-CH₂-COOalkyl zu Verbindungen der Formel HO-CH₂-CH₂-ZR¹-CH₂-CH₂OH (III, X² = X³ = OH) und gegebenenfalls anschließende Umsetzung mit SOCl₂ bzw. PBr₃.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eigenen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente Ind-Q-NH₂ bzw. des Piperidin- oder Piperazinderivates der Formel IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Ferner ist es möglich, eine Verbindung der Formel I zu erhalten, indem man eine Verbindung der Formel Ind-Q-N(CH₂-CH₂-X)₂ (IV) mit einer Verbindung der Formel R¹-NH₂ (V) umsetzt.

Die Verbindungen der Formel V sind zum größten Teil bekannt; die nicht bekannten Verbindungen können leicht in Analogie zu den bekannten hergestellt werden. Sie lassen sich beispielsweise ausgehend von den entsprechend substituierten Nitroverbindungen durch Reduktion in die Amine der Formel V überführen. Die Verbindungen der Formel IV lassen sich durch Umsetzung von Ind-Q-Cl, Ind-Q-Br oder Ind-Q-I mit sekundären Aminen der Formel HN(CH₂-CH₂-X)₂ herstellen.

Die Umsetzung der Verbindungen IV und V verläuft nach Methoden wie sie für die Alkylierung von Aminen aus der Literatur bekannt sind und bereits oben angegeben werden.

Es ist ferner möglich eine Verbindung der Formel I zu erhalten, indem man ein Vorprodukt, das anstelle von Wasserstoffatomen eine oder mehrere reduzierbare Gruppe(n) und/oder eine oder mehrere zusätzliche C-C- und/oder C-N-Bindung (en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen; dabei können gleichzeitig Substituenten in der Gruppe Ind, die in der Ausgangsverbindung enthalten sind, reduziert werden. Vorzugsweise bedient man sich hierzu des nascierenden Wasserstoffs oder komplexer Metallhydride, ferner der Reduktion nach Wolff-Kishner sowie der Reduktionen mit Wasserstoffgas unter Übergangsmetallkatalyse.

Bevorzugte Ausgangsstoffe für die Reduktion entsprechen beispielsweise der Formel VI 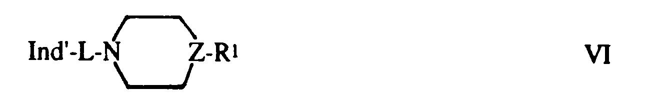 worin
- Ind': einen Rest Ind, der zusätzlich durch eine Arylsulfonylgruppe oder eine Alkyloxycarbonylgruppe in 1-Stellung substituiert sein kann,
- L: Q oder eine dem Rest Q entsprechende Kette, worin jedoch eine oder mehrere -CH₂-Gruppe(n) durch -CO- und/oder ein oder mehrere Wasserstoffatome durch eine oder mehrere OH-Gruppe(n) oder eine Doppelbindung ersetzt sind, bedeuten und
- R¹: die angegebene Bedeutung besitzt
worin jedoch nicht gleichzeitig Ind' = Ind und L = Q sein können.

In den Verbindungen der Formel VI ist L bevorzugt -CO-(CH₂)ₙ₋₂-CO- [im einzelnen -COCO-, -COCH₂CO-, -CO-(CH₂)₂-CO-, -CO-(CH₂)₃-CO-], -(CH₂)ₙ₋₁-CO- [im einzelnen -CH₂-CO-, -CH₂CH₂-CO-, -(CH₂)₃-CO- oder -(CH₂)₄-CO-], ferner z.B. -CO-CH₂CH₂-, -CO-(CH₂)₃-, -CH₂-CO-CH₂CH₂- oder -CH₂CH₂-CO-CH₂-.

Verbindungen der Formel VI sind z.B. herstellbar durch Umsetzung von 4-R¹-piperazin oder -piperidin mit einer Verbindung der Formel VII

Ind'-L-X¹ VII

worin
R¹, Ind', L und X¹ die oben angegebenen Bedeutungen haben, unter den Bedingungen, die zuvor für die Umsetzung von II mit III angegeben sind.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall gelöst in einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerig-alkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie LiAlH₄, NaBH₄, Diisobutylaluminiumhydrid oder NaAl(OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eigenen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit NaBH₄ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden (z.B. solchen der Formel VI, worin L eine -(CH₂)ₙ₋₁-CO-Gruppe bedeutet) mit LiAlH₄ in THF bei Temperaturen zwischen etwa 0 und 66° zu CH₂-Gruppen reduzieren. Dabei können in 1-Stellung des Indolring befindliche Arylsulfonyl-Schutzgruppen gleichzeitig reduktiv abgespalten werden. N-Benzylgruppen können reduktiv mit Natrium im flüssigem Ammoniak abgespalten werden.

Es ist ferner möglich, eine oder mehrere Carbonylgruppen nach der Methode von Wolff-Kishner zu CH₂-Gruppen zu reduzieren, z.B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3-4 Stunden gekocht.

Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Darüber hinaus ist es möglich, bestimmte Reduktionen durch Verwendung von H₂-Gas unter katalytischer Wirkung von Übergangsmetallen, wie z.B. Raney-Ni oder Pd durchzuführen. Man kann auf diese Weise z.B. Cl, Br, I, SH oder in bestimmten Fällen auch OH-Gruppen durch Wasserstoff ersetzen. Ebenso können Nitrogruppen durch katalytische Hydrierung mit Pd/H₂ in Methanol in NH₂-Gruppen umgewandelt werden.

Verbindungen, die sonst der Formel I entsprechen, aber anstelle eines oder mehrerer H-Atome eine oder mehrere solvolysierbare Gruppe(n) enthalten, können zu den Verbindungen der Formel I solvolysiert, insbesondere hydrolysiert werden.

Die Ausgangsstoffe für die Solvolyse sind beispielsweise erhältlich durch Reaktion von IIIa mit Verbindungen, die der Formel II (X¹ = X) entsprechen, aber anstelle eines oder mehrerer H-Atome eine oder mehrere solvolysierbare Gruppe(n) enthalten. So können insbesondere 1-Acylindolderivate (entsprechend der Formel I, aber in 1-Stellung des Ind-Rests eine Acylgruppe enthaltend, vorzugsweise eine Alkoxycarbonyl-, Alkanoyl-, Alkylsulfonyl-oder Arylsulfonylgruppe mit jeweils bis zu 10 C-Atomen, wie Methan-, Benzol- oder p-Toluolsulfonyl) zu den entspechenden in der 1-Stellung des Indolringes unsubstituierten Indolderivaten hydrolysiert werden, z.B. in saurem, besser in neutralem oder alkalischem Medium bei Temperaturen zwischen 0 und 200°. Als Basen verwendet man zweckmäßig Natrium-, Kalium- oder Calciumhydroxid, Natrium- oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Weiterhin kann man eine Verbindung der Formel I nach an sich bekannten Methoden in eine andere Verbindung der Formel I umwandeln.

Verbindungen der Formel I, worin Ind einen durch CO-R¹ substituierten Indol-3-yl-rest bedeutet, können durch Derivatisierung entsprechender Carboxy-indol-3-yl-Verbindungen erhalten werden. Man kann z.B. die Säuren mit entsprechenden Alkoholen oder Alkoholaten unter Verwendung an sich bekannter Methoden verestern. Ferner ist es möglich, Säuren oder Ester mit primären oder sekundären Aminen zu amidieren. Bevorzugt ist die Umsetzung der freien Carbonsäure mit dem Amin unter den Bedingungen einer Peptidsynthese. Diese Reaktion gelingt vorzugsweise in Gegenwart eines Dehydratisierungsmittels, z.B. eines Carbodiimids wie Dicyclohexylcarbodiimid oder N-(3-Dimethylaminopropyl)-N-ethyl-carbodiimid, ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40°, vorzugsweise zwischen 0 und 30°. Anstelle der Säure bzw. des Amids können auch reaktionsfähige Derivate dieser Stoffe in die Reaktion eingesetzt werden, z.B. solche, in denen reaktive Gruppen intermediär durch Schutzgruppen blockiert sind. Die Säuren können auch in Form ihrer aktivierten Ester verwendet werden, die zweckmäßig in situ gebildet werden, z.B. durch Zusatz von 1-Hydroxybenztriazol oder N-Hydroxysuccinimid.

Weiterhin kann man cyan-substituierte Indol-3-yl-reste zu Carboxy-indol-3-yl- oder Carboxamido-indol-3-yl-resten hydrolysieren.

Besonders günstig ist es aber auch in umgekehrter Weise, durch Wasserabspaltung, ausgehend von den Amiden, z.B. mittels Trichloracetylchlorid/Et₃N [Synthesis (2), 184, (1985)] oder mit POCl₃ (J. Org. Chem. 26, 1003 (1961)), die Nitrile herzustellen.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoffe(n) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenden Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierung- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Dabei werden die erfindungsgemäßen Substanzen in der Regel in Analogie zu bekannten, im Handel befindlichen Präparaten (z.B. Bromocriptin, Dihydroergocomin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat filtriert, dampft ein und reinigt durch Chromatographie an Kieselgel und/oder durch Kristallisation. Temperaturen sind in °C angegeben. Rf-Werte wurden dünnschichtchromatographisch an Kieselgel erhalten.

### Beispiel 1

Man löst 1,8 g 3-(4-Chlorbutyl)-5-methoxy-indol [erhältlich durch Diazotierung von p-Methoxyanilin, Umsetzung mit Cyclohexanon-2-carbonsäureethylester nach Japp-Klingemann zu 4-(2-Carbethoxy-indol-3-yl)-buttersäure, Verseifung, Decarboxylierung, Reduktion mit LiAlH₄ und Reaktion mit SOCl₂] sowie 1,9 g 1-(2-Hydroxymethyl-benzofuran-5-yl)-piperazin [erhältlich durch Umsetzung von N,N-Bis-(2-chlorethyl)-amin mit 2-Hydroxymethyl-5-amino-benzofuran] in 200 ml Acetonitril und rührt 10 Stunden bei Raumtemperatur. Nach üblicher Aufarbeitung erhält man 1-[4-(5-Methoxy-indol-3-yl)-butyl]-4-(2-hydroxymethyl-benzofuran-5-yl)-piperazin, F. 159°.

### Analog erhält man durch Umsetzung

von 3-(4-Chlorbutyl)-5-methoxy-indol mit 1-(2,3-Dihydrobenzofuran-5-yl)-piperazin:
1-[4-(5-Methoxy-indol-3-yl)-butyl]-4-(2,3-dihydrobenzofuran-5-yl)-piperazin, F. 111-112°;
von 3-(4-Chlorbutyl)-5-hydroxy-indol mit 1-(Chroman-6-yl)-piperazin:
1-[4-(5-Hydroxy-indol-3-yl)-butyl]-4-(chroman-6-yl)-piperazin, F. 220-222°;
von 3-(4-Chlorbutyl)-5-methoxy-indol mit 1-(Chroman-6-yl)-piperazin:
1-[4-(5-Methoxy-indol-3-yl)-butyl]-4-(chroman-6-yl)-piperazin, F. 129-130°;
von 3-(4-Chlorbutyl)-5-indolcarbonsäuremethylester mit 1-(Chroman-6-yl)-piperazin:
1-[4-(5-Methoxycarbonyl-indol-3-yl)-butyl]-4-(chroman-6-yl)-piperazin;
von 3-(4-Chlorbutyl)-5-indolcarbonsäureethylester mit 1-(Benzo-furan-5-yl)-piperazin:
1-[4-(5-Ethoxycarbonyl-indol-3-yl)-butyl]-4-(benzofuran-5-yl)-piperazin;
von 3-(4-Chlorbutyl)-5-methoxy-indol mit 1-(Benzofuran-5-yl)-piperazin:
1-[4-(5-Methoxy-indol-3-yl)-butyl]-4-(benzofuran-5-yl)-piperazin;
von 3-(4-Chlorbutyl)-5-methoxycarbonyl-indol mit 1-(Chromen-4-on-6-yl)-piperazin:
1-[4-(5-Methoxycarbonyl-indol-3-yl)-butyl]-4-(chromen-4-on-6-yl)-piperazin;
von 3-(4-Chlorbutyl)-5-cyan-indol mit 1-(Chromen-4-on-6-yl)-piperazin:
1-[4-(5-Cyan-indol-3-yl)-butyl]-4-(chromen-4-on-6-yl)-piperazin;
von 3-(4-Chlorbutyl)-5-chlor-indol mit 1-(2,3-Dihydrobenzofuran-5-yl)-piperazin:
1-[4-(5-Chlor-indol-3-yl)-butyl]-4-(2,3-dihydrobenzofuran-5-yl)-piperazin;
von 3-(4-Chlorbutyl)-5-methoxycarbonyl-indol mit 1-(2,3-Dihydrobenzofuran-5-yl)-piperazin:
1-[4-(5-Methoxycarbonyl-indol-3-yl)-butyl]-4-(2,3-dihydrobenzofuran-5-yl)-piperazin;
von 3-(4-Chlorbutyl)-5-methoxycarbonyl-indol mit 4-(2,3-Dihydrobenzofuran-5-yl)-piperidin:
1-[4-(5-Methoxycarbonyl-indol-3-yl)-butyl]-4-(2,3-dihydrobenzofuran-5-yl)-piperidin;
von 3-(4-Chlorbutyl)-5-methoxycarbonyl-indol mit 4-(2,3-Dihydrobenzofuran-5-yl)-4-hydroxy-piperidin:
1-[4-(5-Methoxycarbonyl-indol-3-yl)-butyl]-4-(2,3-dihydrobenzofuran-5-yl)-4-hydroxy-piperidin;
von 3-(4-Chlorbutyl)-5,6-dimethoxy-indol mit 1-(Chroman-6-yl)-piperazin:
1-[4-(5,6-Dimethoxy-indol-3-yl)-butyl]-4-(chroman-6-yl)-piperazin;
von 3-(4-Chlorbutyl)-5-cyan-indol mit 1-(2-Carboxy-benzofuran-5-yl)-piperazin:
1-[4-(5-Cyan-indol-3-yl)-butyl]-4-(2-carboxy-benzofuran-5-yl)-piperazin;
von 3-(4-Chlorbutyl)-6-fluor-indol mit 1-(2,3-Dihydrobenzofuran-5-yl)-piperazin:
1-[4-(6-Fluor-indol-3-yl)-butyl]-4-(2,3-dihydrobenzofuran-5-yl)-piperazin.

### Beispiel 2

Man kocht 1,8 g 1-[4-(5-Methoxycarbonyl-indol-3-yl)-butyl]-4-(chroman-6-yl)-piperazin [erhältlich nach Beispiel 1] 0,5 Std. mit 100 ml 2n ethanolischer KOH, arbeitet wie üblich auf und erhält 1-[4-(5-Carboxy-indol-3-yl)-butyl]-4-chroman-6-yl-piperazin.

Analog erhält man durch Verseifung der entsprechenden Ester ausgehend
von 1-[4-(5-Ethoxycarbonyl-indol-3-yl)-butyl]-4-(benzofuran-5-yl)-piperazin:
1-[4-(5-Carboxy-indol-3-yl)-butyl]-4-(benzofuran-5-yl)-piperazin;
von 1-[4-(5-Methoxycarbonyl-indol-3-yl)-butyl]-4-(chromen-4-on-6-yl)-piperazin:
1-[4-(5-Carboxy-indol-3-yl)-butyl]-4-(chromen-4-on-6-yl)-piperazin;
von 1-[4-(5-Methoxycarbonyl-indol-3-yl)-butyl]-4-(2,3-dihydrobenzofuran-5-yl)-piperazin:
1-[4-(5-Carboxy-indol-3-yl)-butyl]-4-(2,3-dihydrobenzofuran-5-yl)-piperazin;
von 1-[4-(5-Methoxycarbonyl-indol-3-yl)-butyl]-4-(2,3-dihydrobenzofuran-5-yl)-4-hydroxy-piperidin:
1-[4-(5-Carboxy-indol-3-yl)-butyl]-4-(2,3-dihydrobenzofuran-5-yl)-4-hydroxy-piperidin.

### Beispiel 3

2,8 g 1-[4-(5-Carboxy-indol-3-yl)-butyl]-4-(2,3-dihydrobenzo-furan-5-yl)-piperazin werden in 100 ml N-Methylpyrrolidin suspendiert. Anschließend fügt man 3,2 g 2-Chlor-1-methyl-pyridiniummethansulfonat hinzu und rührt bei Raumtemperatur 12 Stunden. In die entstandene Lösung leitet man bis zur Sättigung getrocknetes NH₃-Gas ein und rührt erneut 10 Stunden. Nach üblicher Aufarbeitung erhält man 1-[4-(5-Carbamoyl-indol-3-yl)-butyl]-4-(2,3-dihydrobenzofuran-5-yl )-piperazin.

Analog erhält man durch Amidierung der nachfolgenden Carbonsäuren mit 2-Chlor-1-methyl-pyridiniummethansulfonat:
aus 1-[4-(5-Carboxy-indol-3-yl)-butyl]-4-(2,3-dihydrobenzofuran-5-yl)-piperidin das
1-[4-(5-Carbamoyl-indol-3-yl)-butyl]-4-(2,3-dihydrobenzofuran-5-yl)-piperidin, F. 155-157°;
aus 1-[4-(5-Carboxy-indol-3-yl)-butyl]-4-(2,3-dihydrobenzofuran-5-yl)-4-hydroxy-piperidin das
1-[4-(5-Carbamoyl-indol-3-yl)-butyl]-4-(2,3-dihydrobenzofuran-5-yl-4-hydroxy-piperidin, F. 69° (Zers.);
aus 1-[4-(5-Carboxy-indol-3-yl)-butyl]-4-(chroman-6-yl)-piperazin das
1-[4-(5-Carbamoyl-indol-3-yl)-butyl]-4-(chroman-6-yl)-piperazin.

### Beispiel 4

Analog Beispiel 3 erhält man ausgehend von 1-[4-(5-Cyan-indol-3-yl)-butyl]-4-(2-carboxy-benzofuran-5-yl)-piperazin durch Umsetzung mit 2-Chlor-1-methyl-pyridiniummethansulfonat das 1-[4-(5-Cyan-indol-3-yl)-butyl]-4-(2-carbamoyl-benzofuran-5-yl)-piperazin, F. 269-272° (Hydrochlorid).

### Beispiel 5

Ein Gemisch von 2,6 g 3-(2-Aminoethyl)-5-cyan-indol [erhältlich durch Umsetzung von 5-Cyanindol mit 2-Chloracetylchlorid zu 3-(2-Chloracetyl)-5-cyanindol, anschließende Reduktion mit Diboran, Umsetzung mit Phthalimid und Hydrolyse] und einem Äquivalent 5-[N,N-Bis-(2-chlorethyl)-amino]-benzofuran [erhältlich durch Umsetzung von 2-Chloracetylchlorid mit 5-Aminobenzofuran und anschließende Reduktion mit Diboran] in 40 ml Aceton und 40 ml Wasser wird 20 Stunden gekocht und danach wie üblich aufgearbeitet. Man erhält 1-[2-(5-Cyan-indol-3-yl)-ethyl]-4-(benzofuran-5-yl)-piperazin.

Analog erhält man durch Umsetzung von 5-[N,N-Bis-(2-chlorethyl)-amino]-benzofuran
mit 3-(4-Aminobutyl)-5-methoxymethyl-indol:
1-[4-(5-Methoxymethyl-indol-3-yl)-butyl]-4-(benzofuran-5-yl)-piperazin;
mit 3-(3-Aminopropyl)-5-hydroxy-indol:
1-[3-(5-Hydroxy-indol-3-yl)-propyl]-4-(benzofuran-5-yl)-piperazin;
mit 3-(2-Aminoethyl)-5-methoxy-indol:
1-[2-(5-Methoxy-indol-3-yl)-ethyl]-4-(benzofuran-5-yl)-piperazin;
mit 3-(3-Aminopropyl)-5-indolcarbonsäuremethylester:
1-[3-(5-Methoxycarbonyl-indol-3-yl)-propyl]-4-(benzofuran-5-yl)-piperazin;
mit 3-(2-Aminoethyl)-5-indolcarbonsäureethylester:
1-[2-(5-Ethoxycarbonyl-indol-3-yl)-ethyl]-4-(benzofuran-5-yl)-piperazin;
mit 3-(4-Aminobutyl)-5-fluor-indol:
1-[4-(5-Fluor-indol-3-yl)-butyl]-4-(benzofuran-5-yl)-piperazin.
mit 3-(3-Aminopropyl)-5-cyan-indol:
1-[3-(5-Cyan-indol-3-yl)-propyl]-4-(2-carboxy-benzofuran-5-yl)-piperazin.

### Beispiel 6

Analog Beispiel 5 erhält man durch Umsetzung von 3,2 g 3-(2-Aminoethyl)-5-methoxy-indol mit 1,3 Äquivalenten 6-[N,N-Bis-(2-chlorethyl)-amino]-chroman [erhältlich durch Umsetzung von 2-Chloracetyl-chlorid mit 6-Aminochroman und anschließende Reduktion mit Diboran] das 1-[2-(5-Methoxy-indol-3-yl)-ethyl]-4-(chroman-6-yl)-piperazin.

Analog erhält man durch Umsetzung von 6-[N,N-Bis-(2-chlorethyl)-amino]-chroman
mit 3-(4-Aminobutyl)-5-methoxymethyl-indol:
1-[4-(5-Methoxymethyl-indol-3-yl)-butyl]-4-(chroman-6-yl)-piperazin;
mit 3-(3-Aminopropyl)-5-hydroxy-indol:
1-[3-(5-Hydroxy-indol-3-yl)-propyl]-4-(chroman-6-yl)-piperazin;
mit 3-(2-Aminoethyl)-5-methoxy-indol:
1-[2-(5-Methoxy-indol-3-yl)-ethyl]-4-(chroman-6-yl)-piperazin;
mit 3-(3-Aminopropyl)-5-indolcarbonsäuremethylester:
1-[3-(5-Methoxycarbonyl-indol-3-yl)-propyl]-4-(chroman-6-yl)-piperazin;
mit 3-(2-Aminoethyl)-5-indolcarbonsäureethylester:
1-[2-(5-Ethoxycarbonyl-indol-3-yl)-ethyl]-4-(chroman-6-yl)-piperazin;
mit 3-(4-Aminobutyl)-5-fluor-indol:
1-[4-(5-Fluor-indol-3-yl)-butyl]-4-(chroman-6-yl)-piperazin.
mit 3-(3-Aminopropyl)-5-cyan-indol:
1-[3-(5-Cyan-indol-3-yl)-propyl]-4-(2-carboxy-chroman-6-yl)-piperazin.

### Beispiel 7

Eine Lösung von 3,9 g 1-[4-(5-Carboxy-indol-3-yl)-butyl]-4-(2,3-dihydrobenzofuran-5-yl)-piperazin in 250 ml DMF wird mit 1 g N-Methylmorpholin versetzt. Unter Rühren gibt man eine Lösung von einem Äquivalent tert.-Butylamin in 5 ml DMF, 1,3 g 1-Hydroxybenztriazol sowie eine Lösung von 1,9 g N-(3-Dimethylaminopropyl)-N'-ethyl-carbodimid-hydrochlorid in 20 ml DMF hinzu. Man rührt 16 Stunden bei Raumtemperatur und dampft das Filtrat ein. Nach üblicher Aufarbeitung erhält man 1-[4-(5-N-tert.-Butylcarbamoyl-indol-3-yl)-butyl]-4-(2,3-dihydrobenzofuran-5-yl)-piperazin.

Analog erhält man durch Umsetzung mit tert.-Butylamin ausgehend
von 1-[4-(5-Carboxy-indol-3-yl)-butyl]-4-(chroman-6-yl)-piperazin:
1-[4-(5-N-tert.-Butylcarbamoyl-indol-3-yl)-butyl]-4-chroman-6-yl)-piperazin;
von 1-[4-(5-Cyan-indol-3-yl)-butyl]-4-(2-carboxy-benzofuran-5-yl)-piperazin:
1-[4-(5-Cyan-indol-3-yl)-butyl]-4-(2-N-tert.-butyl-carbamoyl-benzofuran-5-yl)-piperazin.

### Beispiel 8

Eine Gemisch von 2,1 g 1-[4-(5-Methoxy-indol-3-yl)-butyl]-4-(chroman-6-yl)-piperazin [herstellbar nach Beispiel 1], 1,8 g Pyridinhydrochlorid sowie 50 ml Pyridin wird 3 Stunden gekocht. Man kühlt ab, dampft ein, arbeitet wie üblich auf und erhält 1-[4-(5-Hydroxy-indol-3-yl)-butyl]-4-(chroman-6-yl)-piperazin, F. 220-222°.

Analog erhält man
aus 1-[4-(5-Methoxy-indol-3-yl)-butyl]-4-(2,3-dihydrobenzofuran-5-yl)-piperazin:
1-[4-(5-Hydroxy-indol-3-yl)-butyl]-4-(2,3-dihydrobenzofuran-5-yl)-piperazin;
aus 1-[4-(5-Methoxy-indol-3-yl)-butyl]-4-(benzofuran-5-yl)-piperazin:
1-[4-(5-Hydroxy-indol-3-yl)-butyl]-4-(benzofuran-5-yl)-piperazin;
aus 1-[4-(5-Methoxycarbonyl-indol-3-yl)-butyl]-4-(chromen-4-on-6-yl)-piperazin:
1-[4-(5-Hydroxycarbonyl-indol-3-yl)-butyl]-4-(chromen-4-on-6-yl)-piperazin;
aus
1-[4-(5-Methoxymethyl-indol-3-yl)-butyl]-4-(benzofuran-5-yl)-piperazin:
1-[4-(5-Hydroxymethyl-indol-3-yl)-butyl]-4-(benzofuran-5-yl)-piperazin;
aus 1-[2-(5-Methoxy-indol-3-yl)-ethyl]-4-(benzofuran-5-yl)-piperazin:
1-[2-(5-Hydroxy-indol-3-yl)-ethyl]-4-(benzofuran-5-yl)-piperazin;

### Beispiel 9

Analog Beispiel 1 erhält man ausgehend von 3-(4-Chlorbutyl)-5-cyan-indol [erhältlich durch Umsetzung von 5-Cyanindol mit 4-Chlorbutyrylchlorid zu 3-(4-Chlorbutyryl)-5-methoxyindol und anschließende Reduktion mit NaAlH₂(OCH₂CH₂OCH₃)₂] durch Umsetzung mit 1-(2-Ethoxycarbonyl-benzofuran-5-yl)-piperazin [erhältlich durch Umsetzung von N,N-Bis-(2-chlorethyl)-amin mit 2-Ethoxycarbonyl-5-amino-benzofuran] nach üblicher Aufarbeitung das 1-[4-(5-Cyan-indol-3-yl)-butyl]-4-(2-ethoxycarbonyl-benzofuran-5-yl)-piperazin, F. 221-223° (Dihydrochlorid).

Analog erhält man durch Umsetzung
von 3-(4-Chlorbutyl)-5-methoxy-indol mit 1-(2-Cyanbenzofuran-5-yl)-piperazin:
1-[4-(5-Methoxy-indol-3-yl)-butyl]-4-(2-cyanbenzofuran-5-yl)-piperazin;
von 3-(4-Chlorbutyl)-5,6-dimethoxy-indol mit 1-(Chroman-6-yl)-piperazin:
1-[4-(5,6-Dimethoxy-indol-3-yl)-butyl]-4-(chroman-6-yl)-piperazin;
von 3-(4-Chlorbutyl)-5,6-difluor-indol mit 1-(Chroman-6-yl)-piperazin:
1-[4-(5,6-Difluor-indol-3-yl)-butyl]-4-(chroman-6-yl)-piperazin;
von 3-(4-Chlorbutyl)-6-indolcärbonsäuremethylester mit 1-(Chroman-6-yl)-piperazin:
1-[4-(6-Methoxycarbonyl-indol-3-yl)-butyl]-4-(chroman-6-yl)-piperazin;
von 3-(3-Chlorpropyl)-6-indolcarbonsäureethylester mit 1-(2-Cyan-benzofuran-5-yl-piperazin:
1-[3-(6-Ethoxycarbonyl-indol-3-yl)-propyl]-4-(2-cyan-benzofuran-5-yl)-piperazin;
von 3-(4-Chlorbutyl)-5-methoxy-indol mit 1-(2-N-Methylcarbamoyl-benzofuran-5-yl)-piperazin:
1-[4-(5-Methoxy-indol-3-yl)-butyl]-4-(2-N-methylcarbamoyl-benzofuran-5-yl)-piperazin;
von 3-(4-Chlorbutyl)-6-chlor-indol mit 1-(Chromen-4-on-6-yl)-piperazin:
1-[4-(6-Chlor-indol-3-yl)-butyl]-4-(chromen-4-on-6-yl)-piperazin;
von 3-(2-Chlorethyl)-5-cyan-indol mit 1-(Chromen-4-on-6-yl)-piperazin:
1-[2-(5-Cyan-indol-3-yl)-ethyl]-4-(chromen-4-on-6-yl)-piperazin;
von 3-(2-Chlorethyl)-5,6-dichlor-indol mit 1-(2,3-Dihydrobenzofuran-5-yl)-piperazin:
1-[2-(5,6-Dichlor-indol-3-yl)-ethyl]-4-(2,3-dihydrobenzofuran-5-yl)-piperazin;
von 3-(4-Chlorbutyl)-5-methoxycarbonyl-indol mit 1-(2-Carboxy-benzofuran-5-yl)-piperazin:
1-[4-(5-Methoxycarbonyl-indol-3-yl)-butyl]-4-(2-carboxy-benzofuran-5-yl)-piperazin;
von 3-(2-Chlorethyl)-5-methoxycarbonyl-indol mit 4-(2-Carboxy-benzofuran-5-yl)-4-piperidin:
1-[2-(5-Methoxycarbonyl-indol-3-yl)-ethyl]-4-(2-carboxy-benzofuran-5-yl)-piperazin;
von 3-(4-Chlorbutyl)-6-methoxycarbonyl-indol mit 4-(3-Carboxy-benzofuran-5-yl)-4-hydroxy-piperidin:
1-[4-(6-Methoxycarbonyl-indol-3-yl)-butyl]-4-(3-carboxy-benzofuran-5-yl)-4-hydroxy-piperidin;
von 3-(4-Chlorbutyl)-7-methoxycarbonyl-indol mit 4-(3-Carboxy-benzofuran-5-yl)-4-hydroxy-piperidin:
1-[4-(7-Methoxycarbonyl-indol-3-yl)-butyl]-4-(3-carboxy-benzofuran-5-yl)-4-hydroxy-piperidin;
von 3-(4-Chlorbutyl)-5,6-dimethoxy-indol mit 1-(2-Carboxy-benzofuran-5-yl)-piperazin:
1-[4-(5,6-Dimethoxy-indol-3-yl)-butyl]-4-(2-carboxy-benzofuran-5-yl)-piperazin.

### Beispiel 10

Zu einer Suspension von 0.6 g Lithiumaluminiumhydrid in 20 ml THF wird unter Rühren bei Raumtemperatur eine Lösung von 3,6 g 1-[4-(5-Methoxycarbonyl-indol-3-yl)-butyl]-4-(chromen-4-on-6-yl)-piperazin in 40 ml THF tropfenweise zugegeben. Anschließend rührt man eine weitere Stunde bei 25°, fügt 20 ml verdünnte Natronlauge hinzu, filtriert und arbeitet das Filtrat wie üblich auf. Man erhält 1-[4-(5-Hydroxymethyl-indol-3-yl)-butyl]-4-(chromen-4-on-6-yl)-piperazin.

Analog erhält man durch Reduktion
von 1-[4-(5-Methoxycarbonyl-indol-3-yl)-butyl]-4-(chroman-6-yl)-piperazin das
1-[4-(5-Hydroxymethyl-indol-3-yl)-butyl]-4-(chroman-6-yl)-piperazin;
von 1-[4-(5-Methoxycarbonyl-indol-3-yl)-butyl]-4-(benzofuran-5-yl)-piperazin das
1-[4-(5-Hydroxymethyl-indol-3-yl)-butyl]-4-(benzofuran-5-yl)-piperazin;
von 1-[3-(5-Methoxycarbonyl-indol-3-yl)-propyl]-4-(chroman-6-yl)-piperidin das
1-[3-(5-Hydroxymethyl-indol-3-yl)-propyl]-4-(chroman-6-yl)-piperidi n;
von 1-[2-(5-Methoxycarbonyl-indol-3-yl)-ethyl]-4-(chroman-6-yl)-piperidin das
1-[2-(5-Hydroxymethyl-indol-3-yl)-ethyl]-4-(chroman-6-yl)-piperidin.

### Beispiel 11

In eine siedende Lösung von 2,5 g 1-[4-(5-Carboxy-indol-3-yl)-butyl]-4-(2,3-dihydrobenzofuran-5-yl)-piperazin in 50 ml absolutem Methanol wird 2 Stunden HCl-Gas eingeleitet. Anschließend kocht man eine weitere Stunde, arbeitet wie üblich auf und erhält 1-[4-(5-Methoxycarbonyl-indol-3-yl)-butyl]-4-(2,3-dihydrobenzofuran-5-yl)-piperazin.

Analog erhält man durch Veresterung
von 1-[4-(5-Carboxy-indol-3-yl)-butyl]-4-(2,3-dihydrobenzofuran-5-yl)-4-hydroxy-piperidin:
1-[4-(5-Methoxycarbonyl-indol-3-yl)-butyl]-4-(2,3-dihydrobenzofuran-5-yl)-4-hydroxy-piperidin;
von 1-[4-(5-Carboxy-indol-3-yl)-butyl]-4-(chroman-6-yl)-piperazin:
1-[4-(5-Methoxycarbonyl-indol-3-yl)-butyl]-4-(chroman-6-yl)-piperazin;
von 1-[4-(5-Cyan-indol-3-yl)-butyl]-4-(2-carboxy-benzofuran-5-yl)-piperazin:
1-[4-(5-Cyan-indol-3-yl)-butyl]-4-(2-methoxycarbonyl-benzofuran-5-yl)-piperazin.

### Beispiel A: Injektionsgläser

Eine Lösung von 100 g eines Wirkstoffes der Formel I und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 mg eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ x 2 H₂O, 28,48 g Na₂HPO₄ × 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird in Ampullen abgefüllt, unter aseptischen Bedingungen lyophilisiert und steril verschlossen. Jede Ampulle enthält 10 mg Wirkstoff.

## Patentansprüche

1. Piperidin- und Piperazinderivate der Formel I 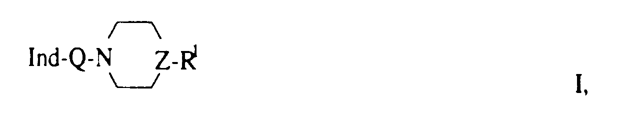 worin
Ind einen unsubstituierten oder einen ein- oder zweifach durch OH, OA, CN, Hal, COR² oder CH₂R² substituierten Indol-3-yl-rest,
R¹ unsubstituiertes oder einfach durch CN, CH₂OH, CH₂OA oder COR² substituiertes Benzofuran-5-yl bzw. 2,3-Dihydrobenzofuran-5-yl, Chroman-6-yl, Chroman-4-on-6-yl, 3-Chromen-6-yl oder Chromen-4-on-6-yl,
Q CₘH₂ₘ,
Z N oder CR³,
A Alkyl mit 1-6 C-Atomen,
Hal F, Cl, Br oder I,
R² OH, OA, NH₂, NHA oder NA₂,
R³ H, OH oder OA und
m 2, 3 oder 4
bedeuten,
sowie deren physiologisch unbedenkliche Salze.

2. (a) 1-[4-(5-Methoxy-indol-3-yl)-butyl]-4-(2-hydroxymethylbenzofuran-5-yl)-piperazin;
(b) 1-[4-(5-Carbamoyl-indol-3-yl)-butyl]-4-hydroxy-4-(2,3-dihydrobenzofuran-5-yl)-piperidin;
(c) 1-[4-(5-Carbamoyl-indol-3-yl)-butyl]-4-(2,3-dihydrobenzofuran-5-yl)-piperidin;
(d) 1-[4-(5-Methoxy-indol-3-yl)-butyl]-4-(2,3-dihydrobenzofuran-5-yl)-piperazin;
(e) 1-[4-(5-Cyan-indol-3-yl)-butyl]-4-(2-ethoxycarbonylbenzofuran-5-yl)-piperazin;
(f) 1-[4-(5-Cyan-indol-3-yl)-butyl]-4-(2-carbamoyl-benzofuran-5-yl)-piperazin;
(g) 1-[4-(5-Methoxy-indol-3-yl)-butyl]-4-(chroman-6-yl)-piperazin;
(h) 1-[4-(5-Hydroxy-indol-3-yl)-butyl]-4-(chroman-6-yl)-piperazin.

3. Verfahren zur Herstellung von Piperazin- und Piperidinderivaten der Formel I nach Anspruch 1 sowie von deren Salzen, dadurch gekennzeichnet, daß man eine Verbindung der Formel II
Ind-Q-X¹ II,
worin
X¹ X oder NH₂ und
X Cl, Br, I, OH oder eine reaktionsfähig funktionell abgewandelte OH-Gruppe bedeuten und
Ind und Q die angegebenen Bedeutungen haben.
mit einer Verbindung der Formel III
X²-(CH₂)₂-ZR¹-(CH₂)₂-X³ III,
worin
X² und X³ gleich oder verschieden sein können und, falls X¹ = NH₂ ist, jeweils X, andernfalls zusammen NH bedeuten und
Z und R¹ die angegebenen Bedeutungen haben,
umsetzt,
oder daß man zur Herstellung einer Verbindung der Formel I, worin Z gleich N bedeutet, eine Verbindung der Formel IV
Ind-Q-N(CH₂-CH₂-X)₂ IV,
worin X, Q und Ind die angegebenen Bedeutungen haben, mit einer Verbindung der Formel V
R¹-NH₂ V,
worin R¹ die angegebene Bedeutung hat,
umsetzt
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
und/oder daß man gegebenenfalls eine OA-Gruppe unter Bildung einer OH-Gruppe spaltet und/oder eine Gruppe Ind und/oder eine Gruppe R¹ in eine andere Gruppe Ind und/oder R¹ umwandelt und/oder daß man eine erhaltene Base oder Saure der Formel I durch Behandeln mit einer Säure oder Base in eines ihrer Salze umwandelt.

4. Verfahren zur Herstellung pharmazeutischer Zubereitungen, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der allgemeinen Formel I gemäß Anspruch 1 und/oder einem ihrer physiologisch unbedenklichen Salze.

6. Verwendung von Verbindungen der Formel I nach Anspruch 1 oder von deren physiologisch unbedenklichen Salzen zur Herstellung eines Arzneimittels.

## Claims

1. Piperidine and piperazine derivatives of the formula I 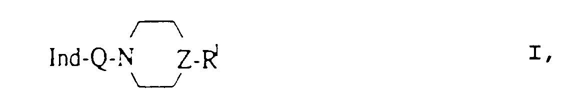 wherein
Ind is an indol-3-yl radical which is unsubstituted or mono- or polysubstituted by OH, OA, CN, Hal, COR² or CH₂R²
R¹ is benzofuran-5-yl or 2,3-dihydrobenzofuran-5-yl, chroman-6-yl, chroman-4-on-6-yl, 3-chromen-6-yl or chromen-4-on-6-yl, which is unsubstituted or mono-substituted by CN, CH₂OH, CH₂OA or COR²,
Q is CₘH₂ₘ,
Z is N or CR³,
A is alkyl having 1-6 C atoms,
Hal is F, Cl, Br or I,
R² is OH, OA, NH₂, NHA or NA₂,
R³ is H, OH or OA and
m is 2, 3 or 4,
and their physiologically acceptable salts.

2. (a) 1-[4-(5-Methoxyindol-3-yl)butyl]-4-(2-hydroxymethylbenzofuran-5-yl)piperazine;
(b) 1-[4-(5-carbamoylindol-3-yl)butyl]-4-hydroxy-4-(2,3-dihydrobenzofuran-5-yl)piperidine;
(c) 1-[4-(5-carbamoylindol-3-yl)butyl]-4(2,3-dihydrobenzofuran-5-yl)piperidine;
(d) 1-[4-(5-methoxyindol-3-yl)butyl]-4-(2,3-dihydrobenzofuran-5-yl)piperazine;
(e) 1-[4-(5-cyanoindol-3-yl)butyl]-4-(2-ethoxycarbonylbenzofuran-5-yl)piperazine;
(f) 1-[4-(5-cyanoindol-3-yl)butyl]-4-(2-carbamoylbenzofuran-5-yl)piperazine;
(g) 1-[4-(5-methoxyindol-3-yl)butyl]-4-(chroman-6-yl)piperazine;
(h) 1-[4-(5-hydroxyindol-3-yl)butyl]-4-(chroman-6-yl)piperazine.

3. Process for the preparation of piperazine and piperidine derivatives of the formula I according to Claim 1, and their salts, characterised in that a compound of the formula II
Ind-Q-X¹ II,
wherein
X¹ is X or NH₂,
X is Cl, Br, I, OH or an OH group functionally modified to form a reactive group, and
Ind and Q are as defined,
is reacted with a compound of the formula III
X²-(CH₂)₂-ZR¹-(CH₂)₂-X³ III,
wherein
X² and X³ can be identical or different and are each X if X¹ = NH₂ or are together NH in other cases, and
Z and R¹ are as defined,
or in that to prepare a compound of the formula I, in which Z is N, a compound of the formula IV
Ind-Q-N(CH₂-CH₂-X)₂ IV,
wherein
X, Q and Ind are as defined, is reacted with a compound of the formula V
R¹-NH₂ V,
wherein
R¹ is as defined,
or in that a compound which has formula I except that one or more hydrogen atoms have been replaced by one or more reducible groups and/or one or more additional C-C and/or C-N bonds is treated with a reducing agent,
or in that a compound which has formula I except that one or more hydrogen atoms have been replaced by one or more solvolysable groups is treated with a solvolysing agent, and/or in that an OA group is optionally cleaved to form an OH group, and/or an Ind group or an R¹ group is converted into another Ind and/or R¹ group, and/or in that a resulting base or acid of the formula I is converted into one of its salts by treatment with an acid or base.

4. Process for the manufacture of pharmaceutical preparations, characterized in that a compound of the formula I according to Claim 1 and/or one of its physiologically acceptable salts are converted into a suitable dosage form together with at least one solid, liquid or semiliquid excipient or adjunct.

5. Pharmaceutical preparation, characterized in that it contains at least one compound of general formula I according to Claim 1 and/or one of its physiologically acceptable salts.

6. Use of compounds of the formula I according to Claim 1, or their physiologically acceptable salts, for the manufacture of a drug.

## Revendications

1. Dérivés de pipéridines et de pipérazines répondant à la formule I 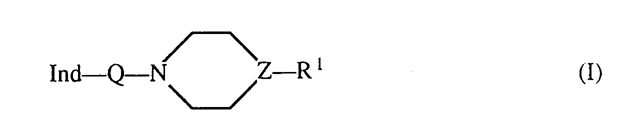 dans laquelle
Ind représente un groupe indole-3-yle non substitué ou portant un ou deux substituants OH, OA, CN, Hal, COR² ou CH₂R²,
R¹ représente un groupe benzofuranne-5-yle ou 2,3-dihydrobenzofuranne-5-yle, chromanne-6-yle, chromanne-4-one-6-yle, 3-chromène-6-yle ou chromène-4-one-6-yle,
Q représente CₘH₂ₘ,
Z représente N ou CR³,
A représente un groupe alkyle en C₁₋₆,
Hal représente F, Cl, Br ou I,
R² représente OH, OA, NH₂, NHA ou NA₂,
R³ représente H, OH ou OA et
m est égal à 2, 3 ou 4,
et leurs sels acceptables pour l'usage pharmaceutique.

2. (a) La 1-[4-(5-méthoxy-indole-3-yl)-butyl]-4-(2-hydroxyméthylbenzofurane-5-yl)-pipérazine ;
(b) la 1-[4-(5-carbamoyl-indole-3-yle)-butyl]-4-hydroxy-4-(2,3-dihydrobenzofuranne-5-yl)-pipéridine ;
(c) la 1-[4-(5-carbamoyl-indole-3-yl)-butyl]-4-(2,3-dihydrobenzofuranne-5-yl)-pipéridine ;
(d) la 1-[4-(5-méthoxy-indole-3-yl)-butyl]-4-(2,3-dihydrobenzofuranne-5-yl)-pipérazine ;
(e) la 1-[4-(5-cyano-indole-3-yl)-butyl]-4-(2-éthoxycarbonylbenzofuranne-5-yl)-pipérazine ;
(f) la 1-[4-(5-cyano-indol-3-yl)-butyl]-4-(2-carbamoylbenzofuranne-5-yl)-pipérazine ;
(g) la 1-[4-(5-méthoxy-indol-3-yl)-butyl]-4-(chromanne-6-yl)-pipérazine ;
(h) la 1-[4-(5-hydroxy-indol-3-butyl]-4-(chromanne-6-yl)-pipérazine.

3. Procédé de préparation de dérivés de pipérazines et de pipéridines de formule I selon la revendication 1 et de leurs sels, caractérisé en ce que l'on fait réagir un composé de formule II
Ind―Q―X¹ (II)
dans laquelle
X¹ représente X ou NH₂ et
X représente Cl, Br, I, OH ou un groupe OH ayant subi une modification fonctionnelle et réactif et
Ind et Q ont les significations indiquées ci-dessus, avec un composé de formule III
X²―(CH₂)₂―ZR¹-(CH₂)₂―X³ (III)
dans laquelle
X² et X³, ayant des significations identiques ou différentes, représentent chacun X lorsque X¹ = NH₂, ou sinon représentent ensemble NH et
Z et R¹ ont les significations indiquées ci-dessus,
ou bien, pour la préparation des composés de formule I dans laquelle Z représente N, on fait réagir un composé de formule IV
Ind―Q―N(CH₂―CH₂―X)₂ (IV)
dans laquelle
X, Q et Ind ont les significations indiquées ci-dessus, avec un composé de formule V
R¹―NH₂ (V)
dans laquelle
R¹ a les significations indiquées ci-dessus,
ou bien on traite par un agent réducteur un composé qui répond à la formule I mais dans laquelle, à la place d'un ou plusieurs atomes d'hydrogène, il y a un ou plusieurs groupes réductibles et/ou une ou plusieurs liaisons C-C et/ou C-N supplémentaires, ou bien on traite par un agent solvolysant un composé répondant à la formule I mais dans laquelle, à la place d'un ou plusieurs atomes d'hydrogène, il y a un ou plusieurs groupes solvolysables,
et/ou on scinde le cas échéant un groupe OA avec formation d'un groupe OH et/ou on convertit un groupe Ind et/ou un groupe R¹ en un autre groupe Ind et/ou R¹ et/ou on convertit un composé de formule I obtenu comme décrit ci-dessus a l'état de base libre ou d'acide libre, en l'un de ses sels, en le traitant par un acide ou par une base.

4. Procédé de préparation de compositions pharmaceutiques caractérisé en ce que l'on met un composé de formule I selon la revendication 1 et/ou l'un de ses sels acceptables pour l'usage pharmaceutique sous une forme de dosage appropriée avec au moins un véhicule ou produit auxiliaire solide, liquide ou semi-liquide.

5. Composition pharmaceutique, caractérisée en ce qu'elle contient au moins un composé de formule générale I selon la revendication 1 et/ou l'un de ses sels acceptables pour l'usage pharmaceutique.

6. Utilisation de composés de formule I selon la revendication 1 ou de leurs sels acceptables pour l'usage pharmaceutique pour la préparation de médicaments.
